# EUROPEAN PATENT APPLICATION

(11) **EP 1 505 392 A1**
(43) Date of publication of application: **09.02.2005**
(21) Application number: 04254300.9
(22) Date of filing: 16.07.2004
(51) Int. Cl.: G01N 33/543, G01N 33/92, B01D 39/00

(54) **Use of lipase for high-density lipoprotein cholesterol detection**

(30) Priority: 16.07.2003 US 487914 P
(71) Applicant: Ortho-Clinical Diagnostics, Inc., Rochester, NY 14626-5101 (US)
(72) Inventor: DiMagno, Theodore John, Penfield, New York 14526 (US); Arter, Thomas Charles, Rochester, New York 14612 (US)
(74) Representative: Mercer, Christopher Paul

(57) **Abstract**

The present invention relates to a dry analytical element useful for the determination and quantification of high-density lipoprotein cholesterol (HDLC) that uses yeast lipase from *Candida rugosa*, a lipasa enzyme source that has cholesterol esterase activity, which preferentially reacts with the cholesterol esters of HDL over cholesterol esters of other lipoproteins.

## Description

### Background

Lipoproteins can be fractionated into high-density lipoproteins (HDL), low-density lipoproteins (LDL), very low-density lipoproteins (VLDL) and chylomicrons (CM). It is known that high-density lipoprotein cholesterol (HDLC) is related to the removal of cholesterol accumulation from tissues including arterial walls. Therefore, HDLC is measured as a negative risk factor for various types of arteriosclerosis such as coronary arteriosclerosis, and the HDLC level in blood is an index useful for the precognition of arteriosclerosis.

The conventional method for determining the amount of HDLC cholesterol consists of two steps, a fractionation step and a detection step. Examples of fractionation include an ultracentrifugation method, an immunochemical method, an electrophoretic method, and a precipitation method. An alternative approach to the conventional solution methods is to perform the HDLC assay in a dry, multilayered, thin-film slide analytical element coated on a polyester support.

The development of the direct HDLC slide requires a preferential selectivity for LDL and VLDL. Many known solution methods have been shown to improve HDLC specificity including non-HDL precipitation methods (1, 2, 3, 4, 5), immuno-inhibition (6, 7), selective surfactants (8, 9, 10), Catalase elimination method (11), and polyethylene glycol (PEG) cross-linked cholesterol esterase (CEH) (12). However, the precipitation methods and selective surfactant methods did not yield sufficient HDLC selectivity in the dry, multilayered slide format. Other methods use multiple reagent additions or separation steps which are not amenable to the all inclusive single-step dry slide technology. Because none of the well known HDLC methods yielded sufficient selectivity in the thin film format, additional methods to improve HDLC specificity were pursued.

Combinations of the known HDLC methods were introduced into the dry slide format to try to improve the HDLC selectivity. Methods coated together in the dry slide format to solve the HDLC selectivity included combinations of different non-HDL precipitating reagents (e.g., phosphotungstic acid, dextran sulfate, polyethylene glycol), HDL selective surfactants, ion exchange resins, LDL complex formers (calix[8]arene (13)), and magnetic particles (14). None of these combinations gave the HDLC specificity performance that was desired, so further methods were explored.

The cholesterol esterase employed in many of the enzymatic reaction sequences is a general esterase that reacts with esters from all lipoprotein sources. A CEH enzyme derived from *Chromobacterium viscosum* was noted to show selectivity for HDLC over LDLC or VLDLC (15), as was the PEG-cross-linked cholesterol esterase noted above. Yet, there remained a desire to find additional CEH sources that show HDLC selectivity that could improve the thin film element performance. The additional selectivity from the potential CEH could be used to improve the inadequate HDLC selectivity obtained using other selective reagents in the direct HDL slide.

### Summary of the Invention

An object of the present invention is to identify an enzyme source that shows selectivity for HDLC and is therefore useful in an assay. A second object is to find an enzyme source that is functional in an HDLC slide assay. A dry analytical element for the quantitation of high-density lipoprotein cholesterol in a sample is disclosed herein based on measuring the change in reflection density at an appropriate wavelength upon formation of dye from the reaction of HDLC in presence of surfactant, cholesterol esterase, and cholesterol oxidase. The dry element of this invention is useful in determining the amount of HDLC in any liquid sample, in particular biological fluids.

Another object of the invention is to provide an analytical element for the determination of the presence of high-density lipoprotein cholesterol comprising a support having thereon at least one reagent layer and containing in said reagent layer a *Candida rugosa* lipase.

Another object of the invention is to provide a kit for determining the presence of high-density lipoprotein cholesterol, containing an analytical element as described above.

Another object of the invention is to provide a method for assaying for a biological sample for the presence of high-density lipoprotein cholesterol comprising providing a support having thereon at least one reagent layer and containing in said reagent layer a *Candida rugosa* lipase; contacting support with a sample that may contain high-density lipoprotein; and detecting the high-density lipoprotein cholesterol.

More specifically, in one embodiment, the present invention relates to a dry analytical element useful for the determination and quantitation of HDLC comprising:
(i) a porous spreading layer,
(ii) one or more additional layers which are in fluid contact with the porous spreading layer, and
(iii) a support,
wherein said element contains, in at least one of the layers, a surfactant which solubilizes high-density lipoprotein, a precipitating reagent for non-HDL, yeast lipase from *Candida rugosa,* cholesterol oxidase, and an indicator dye and horseradish peroxidase which react with the H₂O₂ produced by the enzyme cascade to produce a measurable change in the spectral absorption or reflection density of the dye upon contact of said element with a fluid suspected of comprising HDLC, and wherein said dye, said enzymes, said surfactant, and said non-HDL precipitating reagent may be present together in the same layer or be present in any combination or individually in separate layers of said element is useful in a slide assay.

The present inventors have found that yeast lipase from *Candida rugosa* is a lipase enzyme source that has cholesterol esterase activity which preferentially reacts with the cholesterol esters of HDLC over cholesterol esters of other lipoproteins. The HDLC selectivity observed with this enzyme source in the direct HDLC dry slide format is unusual compared to the lack of selectivity shown by many other screened cholesterol esterase sources. This cholesterol esterase HDLC selectivity adds a third selectivity mechanism to the assay. The additional selectivity from the cholesterol esterase greatly improves the overall selectivity of the assay as a whole and makes it possible to have a functional direct HDLC slide assay. Without this enhancement, the overall selectivity and hence, accuracy of the assay is insufficient due to the interference from non-HDLC lipoproteins.

### Brief Description of the Drawings

Figure 1 shows a kinetic response for human HDL and LDL test fluids with the non-specific porcine pancreas CEH.
Figure 2 shows a kinetic response for human HDL and LDL test fluids with specific *Candida rugosa* lipase.

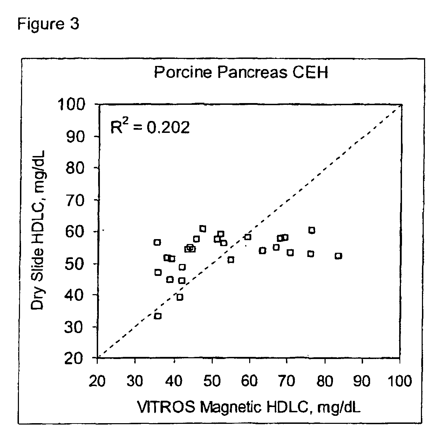

Figure 3 shows a patient accuracy plot using the non-selective porcine pancreas CEH in a direct HDLC slide.

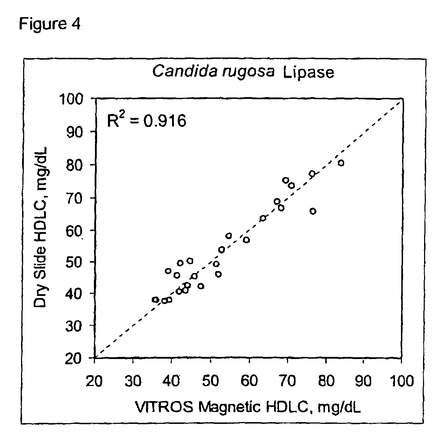

Figure 4 shows a patient accuracy plot using the HDLC selective *Candida rugosa* lipase in a direct HDLC slide.

### Detailed Description of the Invention

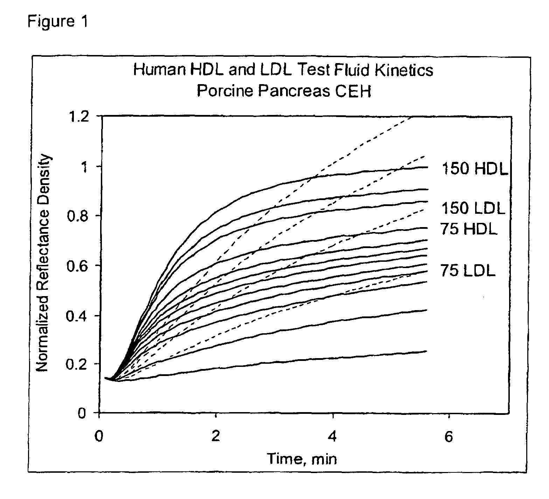

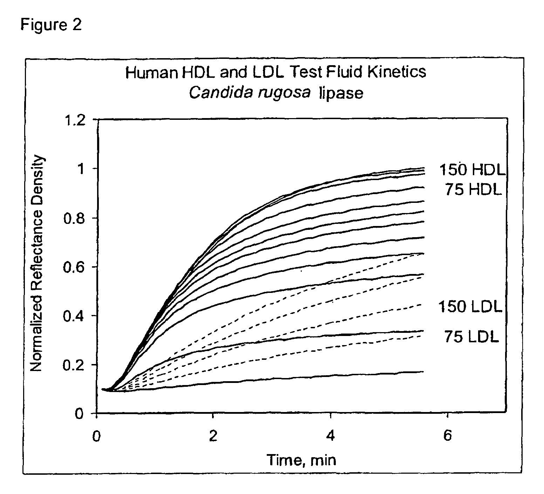

In the direct HDLC dry slide format, a lipase enzyme with cholesterol esterase activity was discovered which showed selectivity for HDLC derived esters over non-HDLC lipoprotein-derived esters. Several esterase and lipase sources were screened for HDLC selectivity, and the HDLC specific cholesterol esterase source was the Lipase II F/D (E.C. 3.1.1.13, *Candida rugosa*) produced by the Genzyme Corporation (catalogue # 70 6551-01). The preferred format of the thin film element used in this evaluation is shown in Example 1, but the locations of the enzymes and other reactive Ingredients may be placed in a variety of positions within the thin film element, and a variety of different materials may be used for the various layers. The HDLC selectivity of this enzyme source was demonstrated by comparing the performance of the *Candida rugosa* Lipase against seven other enzyme sources (Table 2) using two different means: comparison of the kinetic response from pure human HDL and pure human LDL test fluids, and patient accuracy comparisons with neat patient samples. The kinetics of the human LDL fluid at 150 mg/dL compared to the human HDL fluid at 150 mg/dL shows a much larger response for the non-specific HDLC esterase (Figure 1 - Porcine Pancreas CEH) than the response for the HDLC-specific HDLC esterase (Figure 2 *- Candida rugosa* Lipase). Similar non-selectivity was observed with the other esterase and lipase sources summarized in Table 2. The data show that the esterase and lipase sources screened in the HDLC dry slide format have more LDLC reactivity compared to HDLC reactivity than the *Candida rugosa* lipase enzyme.

Dry analytical elements and their use are described in numerous publications, including U.S. Pat. Nos. 4,123,528; 4,786,605; 3,992,158; 4,258,001; 4,670,381; and European Patent Application Nos. 051 183; 066 648. The layers of the element of the present invention can be self-supporting, but preferably, these layers are disposed on a suitable dimensionally stable, chemically inert support. A support of choice should be compatible with the intended mode of detection. Useful support material include but are not limited to paper, metal, foils, polystyrenes, polyesters, polycarbonates, and cellulose esters.

In at least one of the layers of the element of this invention is a dye which is capable of reacting with an enzyme to form a color. In a preferred embodiment of this invention, a leuco dye is used that can react with hydrogen peroxide to form a color. The color can be detected optically by the naked eye, by a photodiode selected to respond to a particular wavelength of light, or by other optical detection systems known by those skilled in the art using absorption, reflectance, or fluorescence spectroscopy. In a preferred embodiment of this invention, a reflectometer is used to detect and quantitate the dye color.

The element of this invention can include a wide variety of additives in appropriate layers as are known in the art to aid in manufacture, fluid spreading, and absorption and unwanted radiation.

The element of the present invention can be prepared using conventional coating procedures and equipment as are described in the art including gravure, curtain, hopper, and other coating techniques. The element can be configured in a variety of forms, including elongated tapes of any desired width, sheets, slides or chips. The process can be manual or automated.

A "sample" as used herein, refers to any substance that may contain the analyte of interest. A sample can be biological fluid, such as whole blood or whole blood components including red blood cells, white blood cells, platelets, serum and plasma, ascites, urine, cerebrospinal fluid, and other constituents of the body which may contain the analyte of interest. Optionally, samples may be obtained from water, soil, and vegetation.

The effectiveness and advantages of the invention are further illustrated by the following examples. The examples are meant to illustrate, but not to limit, the scope and spirit of the invention.

### Example 1

### Example of a multilayer thin film element containing the HDLC-specific cholesterol esterase.

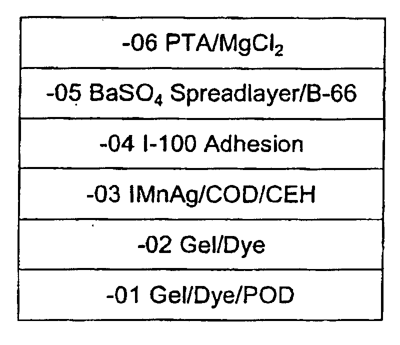

Cholesterol Esterase enzyme sources screened in the thin film element . The term "E.C." as used in

Table 1 enzyme code. It is based on the recommendations of the Nomenclature Committee of the International Union of Biochemistry and Molecular Biology (IUBMB) and it describes each type of characterized enzyme for which an EC (Enzyme Commission) number has been provided.

**Table 1.**

| Vendor | Enzyme | E.C. Number | Source |
|---|---|---|---|
| Toyobo | Lipoprotein Lipase | E.C. 3.1.1.3 | *Pseudomonas sp.* |
| Toyobo | Cholesterol Esterase | E.C. 3.1.1.13 | *Pseudomonas sp.* |
| Asahi | Cholesterol Esterase | E.C. 3.1.1.13 | *Pseudomonas sp.* |
| Asahi | Cholesterol Esterase | E.C. 3.1.1.13 | Microorganism |
| Asahi | Lipoprotein Lipase | E.C. 3.1.1.3 | *Chromobacterium viscosum* |
| Calzyme | Cholesterol Esterase | E.C. 3.1.1.13 | Porcine Pancreas |
| Genzyme | Cholesterol Esterase | E.C. 3.1.1.13 | Beef Pancreas |
| Genzyme | Lipoprotein Lipase | E.C. 3.1.1.3 | *Candida rugosa* |

HDLC selectivity of the *Candida rugosa* lipase and other esterase and lipase enzymes screened (normalized to the *Candida rugosa* lipase; lower number signifies decreased selectivity) are shown in Table 2.

**Table 2.**

| Enzyme Source | Normalized HDLC Selectivity |
|---|---|
| Asahi CEN *Pseudomonas* Esterase | 0.71 |
| Asahi CEBP-M Microorganism Esterase | 0.81 |
| Asahi *Chromobacterium viscosum* Lipase | 0.53 |
| Toyobo *Pseudomonas* Esterase | 0.86 |
| Toyobo *Pseudomonas* Lipase | 0.57 |
| Calzyme Porcine Pancreas Esterase | 0.44 |
| Genzyme Beef Pancreas Esterase | 0.55 |
| Genzyme *Candida rugosa* Lipase | 1.00 |

The *Candida rugosa* lipase is considerably more selective for HDLC lipoproteins when judged with the human HDL and LDL test fluids, and has unique specificity characteristics not seen with other enzymes used in the well known cholesterol enzymatic cascade. The results for the patient accuracy testing show a similar trend as the human HDL and LDL test fluids for the HDLC selectivity. The correlation for the non-specific Porcine Pancreas esterase is considerably worse than the *Candida rugosa* Lipase. The lack of HDLC selectivity results in a very flat slope in the correlation plot and displays a large amount of scatter with the data. With the *Candida rugosa* Lipase in the dry slide, the correlation to the VITROS Magnetic HDLC precipitation method is far superior to the Porcine Pancreas esterase. The cross-reactivity to the non-HDL lipoprotein cholesterol causes the correlation to be very poor for the Porcine Pancreas esterase. Because the *Candida rugosa* Lipase shows HDLC specificity, the cross-reactivity of the non-HDL lipoprotein cholesterol is greatly reduced and the HDLC accuracy is improved. The patient accuracy results for the other screened esterase and lipase sources yield a worse correlation than with the *Candida rugosa* Lipase. The correlation results for the enzyme sources are summarized in Table 3. These results confirm the conclusions reached from the human HDL and LDL test fluids that the *Candida rugosa* Lipase shows HDLC specificity that is not observed with other esterase and lipase sources.

Table 3 shows correlation results between the direct HDLC slide and the VITROS Magnetic HDLC precipitation method.

**Table 3.**

| Enzyme Source | Slope | Intercept | Pooled Bias |
|---|---|---|---|
| Asahi CEN Esterase | 0.71 | 13.7 | 7.6 |
| Asahi CEPB-M Esterase | 0.75 | 11.0 | 7.3 |
| Toyobo Esterase | 0.56 | 20.0 | 7.3 |
| Toyobo Lipase | 0.57 | 23.2 | 9.1 |
| Calzyme Porcine Pancreas Esterase | 0.20 | 42.0 | 12.6 |
| Genzyme Beef Pancreas Esterase | 0.08 | 48.2 | 13.6 |
| Genzyme Lipase II | 0.91 | 4.7 | 4.1 |

The *Candida rugosa* Lipase HDLC specificity discovery improves the overall HDLC specificity of the dry slide element over other esterase and lipase sources. The HDLC specificity improvement is necessary because the currently known HDLC specific methods do not provide adequate HDLC specificity in this dry slide format. This additional increase in specificity in the enzyme cascade allows suitable performance in the dry slide element that would not be possible without this discovery.

All cited materials herein are hereby incorporated by reference. Accordingly, it should be noted that the present invention includes all modifications falling within the scope of the following claims. Specifically, one skilled in the art would understand how to use *Candida rugosa* Lipase in a liquid-based assay system as well.

### Literature Cited

1 Burstein, M., Scholnick, H.R., & Morfin, R. *J Lipid Res,* 1970, **11**, 583-595.
2 Fredrickson, D.S., Levy, R.I., & Lindgren, F.T. *J Clin Invest* 1968, **47**, 2446-2457.
3 Mayfield, C., Warnick, G.R., & Albers, J.J. *Clin Chem* 1979, **25**, 1309-1313.
4Burstein, M., & Scholnick, H.R. *Adv Lipid Res* 1973, **11**, 67-108.
5Briggs, C., Anderson, D., Johnson, P., & Deegan, T. *Ann Clin Biochem* 1981, **18**, 177-181.
6 Kakuyama, T., Kimura, S., & Hasiguchi, Y. *Clin Chem* 1994, **40,** A1104.
7 Nauck, M., März, W., & Wieland, H. *Clin Chem* 1998, **44**, 1443-1451.
8 Harris, N., Galpachian, V., Thomas, J., lannotti, E., Law, T., & Rifai, N. *Clin Chem* 1997, **43,** 816-823.
9 Arranz-Pena, M., Tasende-Mata, J., & Martin-Gil, F.J. *Clin Chem* 1998, **44,** 2499-2505.
10 Yamamoto, M., Nakamura, M., Hino, K., Saito, K., & Manabe, M. *Clin Chem* 2000, **46**, A98.
11 Izawa, S., Okada, M., Matsui, H., & Horita, Y. *J Med Pharm Sci* 1997, **37**, 1385-1388.
12 Sugiuchi, H., Uji, Y., Okabe, H., Irie, T., Uekama, K., Kayahara, N., & Miyauchi, K. *Clin Chem* 1995, **41**, 717-723.
13 Kishi, K., Ochiai, K., Ohta, Y., Uemura, Y., Kanatani, K., Nakajima, K., Wang, T., & Nakamura, M. 69^{th} AACC 2001, Poster, Chicago, IL.
14 Lawlor, J.F., & Musto, J.D. United States Patent 5,242,833, September 7, 1993.
15 Kishi, K., Kakuyama, T., Ochiai, K., & Hasegawa, Y. European Patent EP 1 158 299 A1, February 29, 2000.

## Claims

1. An analytical element for the determination of the presence and quantification of high-density lipoprotein cholesterol comprising a support having thereon at least one reagent layer and containing in said reagent layer a *Candida rugosa* lipase.

2. A kit for determining the presence and quantification of high-density lipoprotein cholesterol comprising an analytical element as claimed in claim 1.

3. A method for assaying for a biological sample for the presence of high-density lipoprotein cholesterol comprising
i) providing a support having thereon at least one reagent layer and containing in said reagent layer a *Candida rugosa* lipase;
ii) contacting support with a sample that may contain high-density lipoprotein; and
iii) detecting and quantifying the high-density lipoprotein cholesterol.
